(19) Europäisches Patentamt
European Patent Office
Office européen des brevets

(11) Numéro de publication: **0 116 801**
**B1**

(12) # FASCICULE DE BREVET EUROPEEN

(45) Date de publication du fascicule du brevet: **01.04.87**

(51) Int. Cl.⁴: **C 08 B 37/10**, A 61 K 31/725

(21) Numéro de dépôt: **83402533.0**

(22) Date de dépôt: **26.12.83**

(54) **Héparine dépolymérisée et supersulfatée, procédé pour sa préparation et compositions pharmaceutiques en contenant.**

(30) Priorité: **28.12.82 FR 8221934**

(43) Date de publication de la demande: **29.08.84 Bulletin 84/35**

(45) Mention de la délivrance du brevet: **01.04.87 Bulletin 87/14**

(84) Etats contractants désignés: **AT BE CH DE FR GB IT LI LU NL SE**

(56) Documents cités:
**FR - A - 2 400 037**
**GB - A - 2 068 011**
**US - A - 2 755 275**

(73) Titulaire: **SCLAVO S.p.A., Via Fiorentina 1, I-53100 Siena (IT)**
Titulaire: **SANOFI, société anonyme, 40, Avenue George V, F-75008 Paris (FR)**

(72) Inventeur: **Naggi, Annamaria, Via Monte Nevoso N. 32, Legnano Milano (IT)**
Inventeur: **Torri, Giangiacomo, Via Confalonieri 8, Bergamo (IT)**

(74) Mandataire: **Gillard, Marie-Louise et al, Cabinet Beau de Loménie 55, Rue d'Amsterdam, F-75008 Paris (FR)**

**EP 0 116 801 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une héparine dépolymérisée et supersulfatée, un procédé pour sa préparation et des compositions pharmaceutiques la renfermant en tant que principe actif.

L'héparine est un polysaccharide constitué par des molécules d'acide glucuronique et d'acide iduronique, éventuellement sulfaté en position 2, associées à des molécules de glucosamine, éventuellement sulfatée en position 6 et sulfatée ou acétylée sur l'amine en position 2.

La structure de l'héparine peut être statistiquement représentée par la formule suivante:

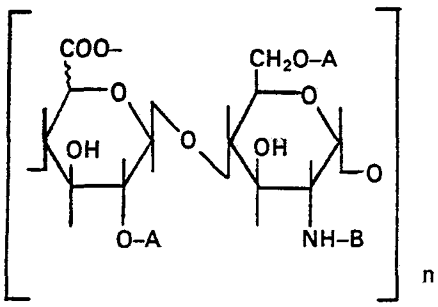

I

où A représente H et $SO_3^-$, B représente $SO_3^-$ et $COCH_3$ et n est compris entre 20 et 30.

L'expression «n est compris entre 20 et 30» indique que la majorité des molécules d'héparine est représentée par la structure I ci-dessus, où l'unité disaccharidique est répétée 20 à 30 fois, ce qui correspond à un poids moléculaire de 12 000 à 18 000.

Les expressions «H et $SO_3^-$» et «$SO_3^-$ et $COCH_3$», telles qu'utilisées ici pour, respectivement, les substituants A et B, indiquent que, dans les 20 à 30 unités disaccharidiques ci-dessus, A est dans certains cas l'hydrogène et dans d'autres cas un groupe $SO_3^-$ et, d'une façon analogue, B est dans la majorité des cas $SO_3^-$ et dans d'autres cas un groupe acétyle.
De même, la liaison , telle qu'indiquée ici, indique que le groupe $COO^-$, dans les 20 à 30 unités disaccharidiques ci-dessus, a dans certains cas la configuration:

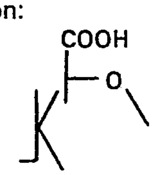

de l'acide D-glucuronique et dans la majorité des n unités la configuration:

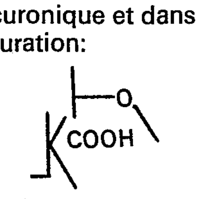

de l'acide L-iduronique.

L'héparine est douée d'une bonne activité antithrombotique, ce qui justifie son emploi thérapeutique actuel, notamment dans la prévention des thromboses veineuses profondes post-chirurgicales. Toutefois, cette activité antithrombotique de l'héparine est largement attribuée à son action anticoagulante et suscite dès lors des problèmes sérieux de surveillance de la part du médecin à cause du haut risque hémorragique associé à l'héparinothérapie.

Il est connu en littérature que, par dépolymérisation de l'héparine jusqu'à des héparines de bas poids moléculaire (par «héparine de bas poids moléculaire», on entend ici des héparines dépolymérisées ayant un poids moléculaire entre 2000 et 9000), on obtient des composés ayant pratiquement la même activité antithrombotique mais un effet anticoagulant amoindri (Seminars in Hematology 1978, 15, 1–17).

Il est aussi généralement reconnu qu'à égalité de degré de polymérisation, l'activité biologique des polysaccharides augmente avec leur degré de sulfatation.

Le terme «degré de sulfatation», dans le cas de l'héparine et, en général, des autres glycosaminoglycanes, désigne le nombre de groupes sulfate ($SO_3^-$) par unité disaccharidique I ci-dessus. Les héparines commerciales pures, généralement obtenues à partir de la muqueuse intestinale de porc, ont un degré de sulfatation compris entre 1,8 à 2,3, en général d'environ 2. Le degré de sulfatation est aussi exprimé par le rapport $SO_3^-/COO^-$.

De nombreux procédés pour la dépolymérisation de l'héparine et l'obtention conséquente d'héparines de bas poids moléculaire ont été décrits en littérature.

La demande de brevet européen publiée sous le N° 37 318 et la demande de brevet internationale n° 81 00 519 décrivent un procédé d'hydrolyse déaminative par action de l'acide nitreux. Ce procédé comporte l'obtention d'une héparine dépolymérisée ayant, à un bout de la chaîne, un aldéhyde ayant le squelette:

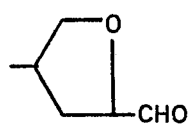

II

La demande de brevet européen publiée sous le n° 40 144 décrit un procédé d'hydrolyse basique qui conduit, par béta-élimination, à des héparines dépolymérisées ayant, à un bout de la chaîne, un sucre insaturé ayant le squelette:

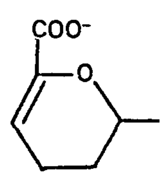

III

La demande de brevet français publiée sous le n° 2 474 508 décrit une hydrolyse acide effectuée avec de l'acide ascorbique et de l'eau oxygénée qui conduit à des héparines dépolymérisées ayant perdu le groupement $SO_3^-$ dans la position 2 de la glucosamine, qui est responsable de l'activité biologique. Le produit ainsi obtenu doit donc être resulfaté pour récupérer son activité.

Un autre procédé connu pour la dépolymérisation de l'héparine (J. Biol. Chem. 1982, 257,

7310–7313) consiste en une hydrolyse enzymatique qui conduit à un produit ayant, à un bout de la chaîne, le sucre insaturé III ci-dessus et ayant perdu 90% de son activité.

Par ailleurs, la demande de brevet EP 83 402 534.8 (EP-A-0 116 251) aux noms des demanderesses et revendiquant la même date de priorité que la présente demande a pour objet un procédé pour la dépolymérisation et la sulfatation des polysaccharides, y compris l'héparine. Ce procédé consiste à faire réagir les polysaccharides avec un mélange d'acide sulfurique et l'acide chlorosulfonique.

On a maintenant trouvé que, par traitement de l'héparine avec un mélange d'acide sulfurique et d'acide chlorosulfonique, on obtient, avec des bons rendements, une héparine dépolymérisée ayant un poids moléculaire entre 2000 et 9000.

On a aussi trouvé d'une façon surprenante que l'héparine dépolymérisée ainsi obtenue possède un degré de sulfatation d'au moins 20% supérieur à celui de l'héparine de départ. Cette nouvelle héparine est ici désignée «supersulfatée».

On a également constaté que, dans l'héparine dépolymérisée et supersulfatée ainsi obtenue tous les hydroxyles primaires, position 6 de la glucosamine, sont estérifiés par un groupe sulfate.

On a finalement trouvé que la nouvelle héparine dépolymérisée et supersulfatée montre une bonne activité fibrinolytique et hypolipémiante associée à une très faible activité anticoagulante.

Ainsi, selon un de ses aspects, la présente invention a pour objet une nouvelle héparine dépolymérisée et supersulfatée ayant un poids moléculaire compris entre 2000 et 9000 et un degré de sulfatation supérieur d'au moins 20% à celui de l'héparine correspondante.

Cette augmentation de degré de sulfatation est donnée en pourcentage car les héparines commerciales ont un degré de sulfatation qui peut varier selon la source et le procédé d'extraction et/ou de purification.

De toute façon, le degré de sulfatation de la nouvelle héparine dépolymérisée et supersulfatée de la présente invention est au moins 2,5, à savoir supérieur à celui de toutes les héparines connues, obtenues de la muqueuse intestinale de porc, et de toutes les héparines de bas poids moléculaire décrites en littérature.

La nouvelle héparine dépolymérisée et supersulfatée de la présente invention est caractérisée par une structure représentée par la formule suivante:

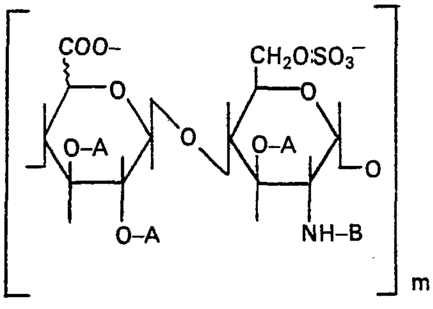

IV

dans laquelle A et B sont tels que définis ci-dessus et m est compris entre 4 et 15.

Comme pour la formule I ci-dessus, les expressions «H et $SO_3^-$» et «$SO_3^-$ et $COCH_3$», utilisées pour, respectivement, A et B, ainsi que le m et la liaison ≀ mettent en évidence le caractère statistique de la formule IV.

Dans les formules I et IV ci-dessus, ainsi que dans les revendications, les produits sont indiqués sous forme anionique. Le cation peut être l'hydrogène, un métal alcalin, de préférence le sodium, ou alcalino-terreux, de préférence le calcium, ou une amine organique physiologiquement compatible.

Selon un autre de ses aspects, la présente invention a pour objet un procédé pour la préparation d'une héparine dépolymérisée et supersulfatée ayant un poids moléculaire entre 2000 et 9000, plus particulièrement représentée par la formule IV ci-dessus, et de ses sels pharmaceutiquement acceptables, caractérisé en ce qu'une héparine d'origine naturelle ou une de ses fractions est traitée avec un mélange d'acide sulfurique et d'acide chlorosulfonique et le produit ainsi obtenu est isolé sous forme de sel alcalin ou transformé dans la forme acide ou dans un autre sel pharmaceutiquement acceptable.

Les deux acides, dans le mélange, sont concentrés; de préférence, leur concentration est au moins 95% en poids.

Le rapport des deux acides est très variable, mais un rapport acide sulfurique: acide chlorosulfonique d'environ 2:1 est particulièrement préféré.

La température de réaction peut varier entre −20 °C et +40 °C; après une période variable de quelques minutes à 2 heures, selon la température de réaction, la dépolymérisation souhaitée est achevée et l'héparine de bas poids moléculaire et supersulfatée est isolée sous forme de sel alcalin en solution aqueuse par précipitation dans un solvant approprié, par exemple l'éther diéthylique ou diisopropylique, dissolution dans l'eau, neutralisation par un hydroxyde alcalin ou un carbonate alcalin, de sodium de préférence, et dialyse finale pour l'élimination des fragments plus petits.

L'héparine dépolymérysée et supersulfatée est isolée sous forme de sel alcalin selon les techniques classiques, par exemple par lyophilisation ou évaporation sous pression réduite, et caractérisée par les méthodes physico-chimiques connues.

D'autres sels, par exemple le sel de calcium, peuvent être obtenus à partir des sels alcalins, le sel de sodium de préférence, par réaction d'échange avec le sel approprié, par exemple un sel de calcium, en utilisant éventuellement une résine échangeuse d'ions.

Les méthodes physico-chimiques employées ont montré que les nouvelles héparines dépolymérisées et supersulfatées de la présente invention sont qualitativement différentes des héparines correspondantes et de toutes les héparines dépolymérisées connues car, à égalité de degré de polymérisation, leur profil de sulfatation est significativement différent à cause des groupes sulfate additionnels.

Une telle différence est mise en évidence aussi bien par le comportement électrophorétique que par les caractéristiques des spectres RMN.

Selon la technique de l'électrophorèse dans l'acétate de baryum, la migration des espèces sulfatées est inversement proportionnelle à leur capacité de complexer les ions $Ba^{++}$. Cette capacité complexante est fonction aussi bien du poids moléculaire que de la densité de charge.

Dans le cas de l'héparine non modifiée, les chaînes ayant une plus grande affinité pour le baryum s'arrêtent (espèce «slow-moving») tandis que les autres migrent vers l'anode (espèce «fast-moving»).

Dans le cas des héparines dépolymérisées connues, on n'observe que des espèces «fast-moving».

Au contraire, et à la différence des héparines naturelles, les nouvelles héparines dépolymérisées et supersulfatées de la présente invention ne montrent que des espèces «slow-moving».

Les spectres RMN confirment la différence qualitative entre les héparines commerciales ou les héparines dépolymérisées connues (dont les spectres sont substantiellement identiques, sauf pour ce qui concerne les signaux des groupes terminaux) et les héparines dépolymérisées et supersulfatées de la présente invention. En effet, les spectres RMN de ces dernières présentent des déplacements significatifs des signaux qui peuvent être attribués à des groupes sulfate nouveaux introduits dans les positions normalement non sulfatées. De tels spectres RMN justifient la formule IV ci-dessus.

Le procédé de la présente invention peut être conduit non seulement sur des héparines commerciales, mais aussi sur des fractions de telles héparines pour obtenir les nouvelles héparines dépolymérisées et supersulfatées de la présente invention.

Selon le procédé de la présente invention, la dépolymérisation a lieu dans des conditions qui ne provoquent pas de variation structurelle dans les unités saccharidiques, notamment sans que l'on obtienne des produits insaturés ou aldéhydiques et pratiquement sans provoquer de décarboxylation.

Les nouvelles héparines dépolymérisées et supersulfatées de la présente invention ont été évaluées chez le rat dans les tests de coagulation sanguine, de fibrinolyse et sur le test de l'activité lipoprotéine lipase circulante.

L'action des composés de la présente invention sur la coagulation sanguine a été quantifiée par le rapport entre l'activité vis-à-vis du facteur Xa (action anti-Xa) et celle sur la coagulation globale extrinsèque (APTT; Activated Partial Thromboplastin Time).

Le facteur Xa est l'enzyme responsable de la transformation de prothrombine en thrombine; l'action anti-Xa prévient donc l'apparition de thrombine circulante. L'action sur l'APTT tient compte de tous les effets vis-à-vis de l'ensemble des facteurs de la coagulation participant à la voie extrinsèque y compris la thrombine, il est donc considéré comme une mesure indirecte du risque hémorragique particulièrement sous héparinothérapie.

Le rapport anti-Xa/APTT permet donc de juger de la composante anticoagulante de l'activité antithrombotique potentielle des héparines dépolymérysées et supersulfatées de la présente invention sans risques hémorragiques associés.

L'autre composante de cette activité antithrombotique potentielle est la fibrinolyse.

L'activité hypolipémiante des composés de la présente invention a été déterminée en évaluant leur action sur la lipoprotéine lipase qui permet d'accélérer le catabolisme des triglycérides.

Trois composés représentatifs de la présente invention, les héparines dépolymérisées et supersulfatées, désignées respectivement par leur numéro de code AH-16 (exemple 1), AH-17 (exemple 4) et AH-19 (exemple 3), ainsi qu'une héparine de départ (D-212/A de l'exemple 4) en tant que produit de référence, ont été administrés chez le rat par la voie intraveineuse à une dose unique de 50 UI/kg (0,3 mg/kg), et les divers paramètres biologiques ont été déterminés 15 minutes après l'administration des produits. L'action sur la coagulation sanguine globale a été mesurée sur des échantillons de plasma citraté selon la technique usuelle (R.R. Proctor and S.I. Rapaporti, Am. J. Clin. Pathol., 1961, 36, 212). Ces mêmes échantillons ont été utilisés pour la détermination de l'activité anti-Xa selon un dosage chronométrique (E.T. Yin, S. Wessler, J.V. Butler, J. Lab. Clin. Med., 1973, 81, 298).

Le tableau I résume l'action des composés de la présente invention sur la coagulation sanguine, en comparaison avec une héparine de départ.

*TABLEAU I*

| Produit | Dose UI/kg i.v. | APTT U/ml | PARAMETRES Anti-Xa U/ml Chronométrique | Anti-Xa / APTT |
|---|---|---|---|---|
| AH-16 | 50 | 0,06 ± 0,02 | 0,18 ± 0,01 | 3 |
| AH-17 | 50 | 0,05 ± 0,02 | 0,17 ± 0,01 | 3,4 |
| AH-19 | 50 | 0,05 ± 0,03 | 0,22 ± 0,04 | 4,4 |
| D-212/A | 50 | 0,212 ± 0,04 | 0,324 ± 0,04 | 1,61 |

Dans un autre essai, deux échantillons d'héparine dépolymérisée et supersulfatée selon la présente invention, AH-104 (exemple 6) et AH-106 (exemple 9) ont été comparés, selon le même test que ci-dessus, à l'héparine de départ D-212 des exemples 1, 3 et 5 à 10. Les produits ont été administrés à la dose de 125 UI/kg (0,75 mg/kg). Les résultats sont consignés dans le tableau II.

*TABLEAU II*

| Produit | Dose UI/kg i.v. | PARAMETRES APTT U/ml | Anti-Xa U/ml Chronométrique | Anti-Xa / APTT |
|---|---|---|---|---|
| AH-104 | 125 | 0,078 ± 0,007 | 0,30 ± 0,03 | 3,84 |
| AH-106 | 125 | 0,080 ± 0,007 | 0,31 ± 0,05 | 3,87 |
| D-212 | 125 | 1,000 ± 0,090 | 1,20 ± 0,04 | 1,20 |

Des tableaux I et II, il ressort que les échantillons AH-104 et AH-106, qui sont identiques, sont plus actifs vis-à-vis du facteur Xa (activité anti-Xa) que sur la coagulation globale. Ils montrent un rapport anti-Xa/APTT au moins double de celui des héparines D-212/A et D-212 de référence.

L'action fibrinolytique a été appréciée par la mesure de l'aire de lyse provoquée par les euglobulines plasmatiques sur des plaques de fibrine (C. Kluft, Haemostasis, 1976, 5, 136). Dans ce cas, la dose administrée en i.v. a été de 0,75 mg/kg.

L'activité lipoprotéine lipase est mesurée par la capacité d'hydrolyser le substrat 14C-trioléine en acide 14C-oléique selon la technique de Nikkola et col. (Metabolism, 1977, 26 (2), 179).

Le tableau III résume l'action ex-vivo d'un composé de la présente invention, le AH-16, sur la fibrinolyse et la lipoprotéine lipase en comparaison avec l'héparine de départ D-212.

*TABLEAU III*

| Produit | PARAMETRES Fibrinolyse (a) aire de lyse | Lipoprotéine lipase (b) % libération |
|---|---|---|
| AH-16 | 304,26 $mm^2$ ± 12,63 (*) | 95 * |
| D-212 | 272,83 $mm^2$ ± 15,43 (**) | 28 |

(a) * $p < 0,01$ ** $p < 0,05$ Test de Duncan; dose administrée 0,75 mg/kg soit 125 UI/kg

(b) Analyse statistique: test de Student comparativement à l'héparine

* $p < 0,01$

De ce tableau, il ressort qu'une augmentation statistiquement significative de l'aire de lyse sur plaque de fibrine a été obtenue pour les échantillons d'euglobulines plasmatiques des animaux traités avec l'héparine standard (D-212). Cette augmentation a été par ailleurs plus prononcée pour les animaux traités avec l'héparine dépolymérisée et supersulfatée (AH-16).

Les résultats obtenus sur la lipoprotéine lipase témoignent d'une activité significative pour le composé de l'invention, de l'ordre de 3 fois supérieure à celle de l'héparine D-212 dont l'activité n'est pas significative.

Les héparines dépolymérisées et supersulfatées de la présente invention sont donc non seulement des antithrombotiques potentiels sans risques hémorragiques associés, mais également des agents hypolipémiants potentiels.

Ainsi, la présente invention a pour objet, selon un autre de ses aspects, des compositions pharmaceutiques à action antithrombotique potentielle, hypolipémiante et fibrinolytique renfermant, en tant que principe actif, une héparine dépolymérisée et supersulfatée de formule IV ci-dessus.

Dans les compositions de la présente invention pour l'administration orale, sublinguale, sous-cutanée, intramusculaire, intraveineuse, transdermique ou rectale, les ingrédients actifs de la formule IV ci-dessus peuvent être administrés sous formes unitaires d'administration, en mélange avec des supports pharmaceutiques classiques, aux animaux et aux êtres humains dans les cas de hausse pathologique de la thrombine et des lipides, notamment dans la prévention des maladies thrombotiques et le traitement de l'athérosclérose.

Parmi les formes unitaires d'administration appropriées, il y a les formes par voie orale, telles que les comprimés, les gélules, les poudres, les granules et les solutions ou suspensions orales et les formes d'administration parentérale utiles pour une administration sous-cutanée, intramusculaire ou intraveineuse.

Afin d'obtenir l'effet antithrombotique et hypolipémiant désiré chez les mammifères et l'homme,

la dose de principe actif peut varier entre 0,1 et 100 mg par kg de poids du corps et par jour.

Chaque dose unitaire peut contenir de 1 à 1500 mg d'ingrédient actif en combinaison avec un support pharmaceutique. Cette dose unitaire peut être administrée 1 à 4 fois par jour pour le traitement des troubles du métabolisme des lipides et en général pour le traitement de la maladie athérosclérotique.

Les exemples suivants illustrent l'invention sans toutefois la limiter.

Exemple 1

A un mélange de 20 ml d'acide sulfurique à 95% et 10 ml d'acide chlorosulfonique, refroidi entre −4 et 0 °C, on ajoute 1 g d'héparine provenant d' muqueuse intestinale de porc (PROQUIFIN lot 7926–7935, numéro de code: D-212) ayant un degré de sulfatation de 1,95 et un poids moléculaire (P.M.) de 13 500, puis on abandonne le mélange réactionnel une heure à la même température sous agitation et encore 60 minutes à la température ambiante. On verse le mélange dans 500 ml d'éther diéthylique froid (−4 à +4 °C), on filtre le précipité et on le lave avec de l'éther diéthylique froid. On dissout le produit ainsi obtenu dans l'eau, on le neutralise par de l'hydroxyde de sodium 0,5 N et on le dialyse contre l'eau distillée dans des membranes à 3500 D (THOMAS DIALYZER TUBING 3787–H 47, diamètre de 11 mm). On obtient ainsi un dessalement ainsi que l'élimination des fragments à plus bas poids moléculaire. Par évaporation lente sous pression réduite, on obtient, avec un rendement de 93% en poids, une héparine sodique dépolymérisée et supersulfatée (n° de code AH-16) sous forme de poudre ayant les caractéristiques suivantes:

– P.M. environ 6000. «(Formule IV, m ≃ 9)».

– Analyse élémentaire: S: 12,93%; C: 18,48%; H: 3,30%; N: 1,76%.

– Degré de sulfatation (rapport $SO_3^-/COO^-$): 3,0.

– Spectre IR: large bande dans la région 1300–1200 $cm^{-1}$, caractéristique des groupes sulfates.

– Electrophorèse en acide chlorhydrique: dans cette technique, la migration est fonction du degré de sulfatation. La figure 1 montre l'augmentation significative de la migration électrophorétique de l'héparine dépolymérisée et supersulfatée par rapport à l'héparine de départ.

– Electrophorèse en acétate de baryum. La figure 2 montre que l'héparine dépolymérisée et supersulfatée a une caractéristique électrophorétique «slow-moving», à la différence de l'héparine de départ qui contient aussi bien les espèces «slow-moving» que les «fast-moving».

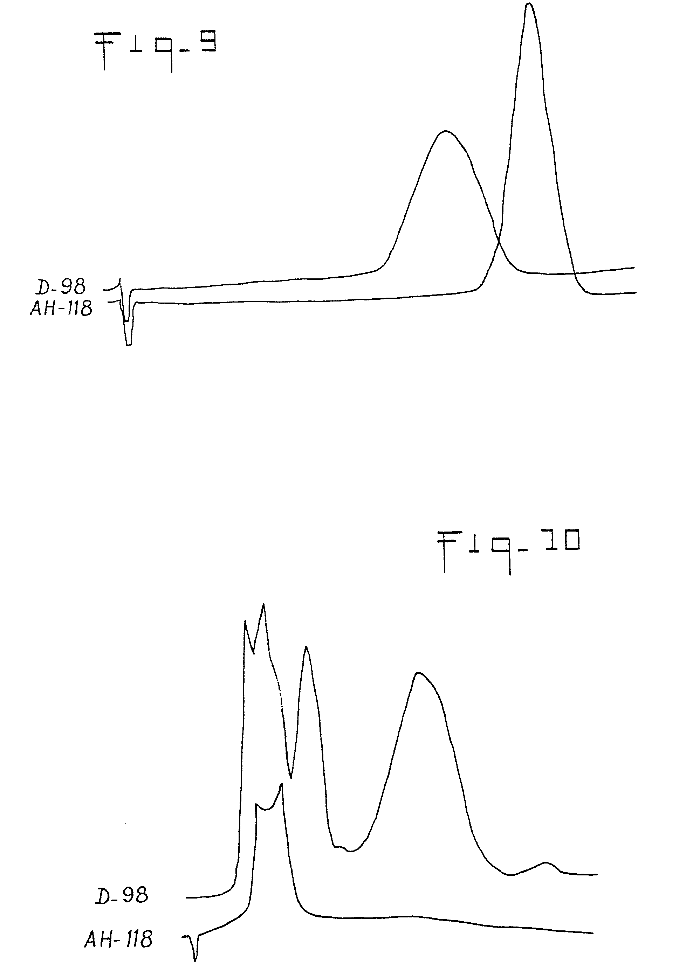

– Spectre 13C-RMN: la figure 3 montre la comparaison des spectres de l'héparine de départ et de l'héparine dépolymérisée et supersulfatée obtenue. Dans le spectre de la nouvelle héparine de bas poids moléculaire, on note l'apparition de nouveaux signaux dûs à l'effet de la dépolymérisation et de l'introduction de groupes sulfates additionnels ainsi qu'à la disparition du signal 6-OH. L'héparine dépolymérisée et supersulfatée ainsi obtenue présente un degré de sulfatation supérieur de 53% à celui de l'héparine de départ sans qu'il y ait eu de décarboxylation significative.

Exemple 2

A un mélange de 10 ml d'acide sulfurique à 98% et 5 ml d'acide chlorosulfonique, refroidi entre −4 et 0 °C, on ajoute 500 mg d'une fraction de poids moléculaire élevé (P.M. 16 500, numéro de code: D-212/B), obtenue par précipitation avec éthanol et ayant un degré de sulfatation $SO_3^-/COO^- = 2$, de l'héparine PROQUIFIN, lot 7926–7935. On abandonne le mélange une heure à la température ambiante, puis on le verse dans 250 ml d'éther diéthylique froid (de −10 à +4 °C), on le filtre, on dissout le précipité obtenu dans de l'eau, on neutralise la solution ainsi obtenue avec de l'hydroxyde de sodium 0,5 M et on dialyse contre de l'eau distillée dans des membranes à 3500 D (THOMAS DIALYZER TUBING 3787-H47) ayant un diamètre de 11 mm afin d'éliminer les sels et les produits de réaction de taille plus petite.

Par évaporation sous pression réduite, on obtient, avec un rendement de 60%, le sel sodique d'une héparine dépolymérisée et supersulfatée (n° de code: AH-18), ayant les caractéristiques suivantes:

– P.M. 3000–5000. «(Formule IV, m = 5–8)».

– Analyse élémentaire: S: 13,56%; C: 18,03%; H: 3,00%; N: 1,70%.

– Degré de sulfatation (rapport $SO_3^-/COO^-$): 2,6.

– Spectre IR: large bande dans la région 1300–1200 $cm^{-1}$, caractéristique des groupes sulfates.

– Electrophorèse en acétate de baryum: la figure 4 montre que l'AH-18 ne montre que les espèces «slow-moving», tandis que le produit de départ montre aussi des espèces «fast-moving».

Exemple 3

A un mélange de 10 ml d'acide sulfurique à 98% et 5 ml d'acide chlorosulfonique, refroidi entre −4 et 0 °C, on ajoute 500 mg du sel sodique d'une héparine provenant de muqueuse intestinale de porc (PROQUIFIN, lot 7926–7935; numéro de code: D-212) ayant un degré de sulfatation $SO_3^-/COO^- = 1,95$. On abandonne le mélange une heure à la température ambiante, puis on le verse dans 250 ml d'éther diéthylique froid (de −10 à +4 °C) et on le traite ensuite comme décrit aux exemples 1 et 2. On obtient ainsi, avec un rendament de 90%, une héparine sodique dépolymérisée et supersulfatée (n° de code: AH-19), ayant les caractéristiques suivantes:

– P.M. environ 6000. «(Formule IV, m = 9)».

– Degré de sulfatation (rapport $SO_3^-/COO^-$): 3.

– Spectre IR: large bande dans la région 1300–1200 $cm^{-1}$, caractéristique des groupes sulfates.

– Electrophorèse en acétate de baryum: la figure

5 montre que l'AH-19 ne montre que des espèces «slow-moving», tandis que le produit de départ montre également des espèces «fast-moving».

Exemple 4

A un mélange de 10 ml d'acide sulfurique à 98% et 5 ml d'acide chlorosulfonique à 95%, refroidi entre −4 et 0 °C, on ajoute 500 mg d'une fraction héparinique de poids moléculaire moyen (P.M. environ 10 000, numéro de code: D-212/A), obtenue par fractionnement avec l'éthanol de l'héparine PROQUIFIN lot 7926–7935, ladite fraction ayant un degré de sulfatation $SO_3^-/COO^-$ de 1,5 et un profil électrophorétique dans l'acétate de baryum qui montre un composant «fast-moving» très important. On abandonne le mélange une heure sous agitation à la température ambiante, puis on le verse dans 250 ml d'éther diéthylique froid (de −10 à +4 °C) et on le traite ensuite comme décrit aux exemples 1 et 2. On obtient ainsi une héparine sodique dépolymérisée et supersulfatée (n° de code: AH-17), ayant les caractéristiques suivantes:

- P.M. 3000–5000. «(Formule IV, m = 5–8)».
- Analyse élémentaire: S: 12,70%; C: 17,24%; H: 3,10%; N: 1,67%.
- Degré de sulfatation rapport $SO_3^-/COO^-$): 2,5.
- Spectre IR: large bande dans la région 1300–1200 $cm^{-1}$, caractéristique des groupes sulfates.
- Electrophorèse en acétate de baryum: la figure 6 montre que l'AH-17, comparé à la fraction héparinique de départ, ne montre que le composant «slow-moving».

Exemple 5

A un mélange de 20 ml d'acide sulfurique à 95% et 10 ml d'acide chlorosulfonique à 98%, refroidi à une température entre −4 et 0 °C, on ajoute 1 g d'héparine provenant de muqueuse intestinale de porc (PROQUIFIN, lot 7926–7935, numéro de code: D-212) ayant un degré de sulfatation de 1,95, puis on abandonne le mélange réactionnel une heure à la température ambiante sous agitation. On verse le mélange dans 500 ml d'éther diéthylique froid (−4 à +4 °C), on filtre le précipité et on le lave avec de l'éther diéthylique froid. On dissout le produit ainsi obtenu dans une solution aqueuse de chlorure de calcium 0,1 M, on ajoute à la solution ainsi obtenue de l'hydroxyde de calcium 0,5 M jusqu'à pH 8 et on dialyse d'abord contre 500 ml d'une solution de chlorure de calcium 0,1 M et ensuite contre de l'eau distillée. Par évaporation lente sous pression réduite, on obtient le sel de calcium d'une héparine dépolymérisée et supersulfatée, sous forme de poudre blanche.

Exemples 6 à 10

A un mélange de 10 ml d'acide sulfurique à 98% et 5 ml d'acide chlorosulfonique, refroidi à une température entre −4 et 0°C, on ajoute 500 mg d'héparine provenant de muqueuse intestinale de porc (PROQUIFIN, lot 7926–7935, numéro de code: D-212) ayant un degré de sulfatation de 1,95 et un poids moléculaire de 13 500. En opérant comme décrit dans l'exemple 1, on obtient avec un rendement de 98% une héparine dépolymérisée et supersulfatée (numéro de code: AH-104).

Le même procédé a été utilisé dans 4 essais parallèles sur la même héparine de départ. On a obtenu les composés désignés par les numéros de code AH-103, AH-105, AH-106, AH-107. Les caractéristiques des 5 produits sont consignés dans le tableau IV.

*TABLEAU IV*

| Ex. | Produit | Analyse élémentaire S % | C % | H % | N % | Degré de sulfatation | Rendement en poids |
|---|---|---|---|---|---|---|---|
| 6 | AH-104 | 14,54 | 15,42 | 2,84 | 1,43 | 2,9 ± 0,1 | 98% |
| 7 | AH-103 | 14,63 | 15,53 | 2,76 | 1,43 | 2,8 ± 0,1 | 89% |
| 8 | AH-105 | 14,48 | 15,43 | 2,61 | 1,44 | 3,0 ± 0,1 | 67% |
| 9 | AH-106 | 14,54 | 15,53 | 2,81 | 1,46 | 2,8 ± 0,1 | 96% |
| 10 | AH-107 | 14,12 | 15,65 | 2,80 | 1,40 | 3,0 ± 0,1 | 77% |

- Poids moléculaire: environ 6000 pour les 5 produits.
- Spectre IR: pour les 5 produits, identique à celui du composé AH-16 décrit dans l'exemple 1.
- Electrophorèse en HCl: tracés identiques à ceux de la figure 1, aussi bien pour le produit de départ que pour les 5 produits obtenus.
- Electrophorèse en acétate de baryum: les profils électrophorétiques sont identiques à ceux de la figure 2 aussi bien pour l'héparine de départ que pour les 5 produits, sauf le fait que les tracés des 5 produits ne contiennent pas les bruits de fond que l'on observe dans la partie horizontale du tracé relatif au produit AH-16 (figure 2), dûs à un défaut de la plume et du papier.
- Spectre 13C-RMN: pour les 5 produits et pour le composé de départ, tracés identiques à ceux de la figure 3.

Les 5 composés ainsi obtenus sont identiques entre eux et également identiques au composé décrit dans l'exemple 1.

Exemples 11 à 14

Dans quatre essais parallèles, on ajoute, à un

mélange de 10 ml d'acide sulfurique à 98% et 5 ml d'acide chlorosulfonique, refroidi entre −4 et 0 °C, 500 mg d'héparine provenant de muqueuse intestinale de porc (DIOSYNTH, batch CH/N 665, numéro de code: D-479), préalablement lyophilisée, ayant un degré de sulfatation ($SO_3^-/COO^-$) de 2,1 et un poids moléculaire d'environ 11 000. On abandonne le mélange réactionnel 1 heure à 0 °C, puis on le verse dans 250 ml d'éther diéthylique préalablement refroidi (entre −10 °C et +4 °C). En opérant ensuite comme décrit dans l'exemple 1, on obtient les produits indiqués dans le tableau V.

*TABLEAU V*

| Ex. | Produit | Analyse élémentaire | | | | Degré de sulfatation | Rendement en poids |
|---|---|---|---|---|---|---|---|
| | | S % | C % | H % | N % | | |
| 11 | AH-108 | 14,88 | 15,29 | 2,52 | 1,47 | 3,1 ± 0,1 | 90% |
| 12 | AH-109 | 14,43 | 15,48 | 2,72 | 1,44 | 3,0 ± 0,1 | 106% |
| 13 | AH-110 | 14,45 | 15,72 | 2,76 | 1,50 | 2,9 ± 0,1 | 65% |
| 14 | AH-111 | 14,55 | 15,08 | 2,60 | 1,41 | 3,0 ± 0,1 | 23% |

– Poids moléculaire: environ 6000 pour les 4 produits.

– Spectre IR: large bande entre 1300 et 1200 $cm^{-1}$, caractéristique des groupes sulfates.

– Electrophorèse en HCl: la fiugre 7 montre les tracés relatifs à l'héparine de départ D-479 et à un des 4 échantillons obtenus dans les différents essais (AH-108). Ceux des trois autres composés sont identiques. Cette figure met en évidence l'augmentation significative de la migration électrophorétique de l'héparine dépolymérisée et supersulfatée par rapport à l'héparine de départ.

– Electrophorèse en acétate de baryum: la figure 8 montre les tracés relatifs à l'héparine de départ D-479 et à AH-108. On peut constater que l'héparine dépolymérisée et supersulfatée a une caractéristique électrophorétique «slow-moving», à la différence de l'héparine de départ qui contient aussi bien les espèces «slow-moving» que les espèces «fast-moving». Les tracés relatifs aux produits AH-109, AH-110 et AH-111 sont identiques à celui de AH-108.

Exemple 15

A un mélange de 10 ml d'acide sulfurique à 98% et 5 ml d'acide chlorosulfonique, refroidi entre −4 et 0 °C, on ajoute 500 mg d'héparine provenant de muqueuse intestinale de porc (THERHORMON batch 575/018, numéro de code: D-98) ayant un poids moléculaire de 13 500 et un degré de sulfatation de 1,8.

En opérant comme décrit dans l'exemple 1, on obtient, avec un rendement de 75% en poids, une héparine dépolymérisée et supersulfatée ayant les caractéristiques suivantes:

– Poids moléculaire: environ 6000.

– Analyse élémentaire: S: 13,90%; C: 15,75%; H: 2,96%; N: 1,48%.

– Degré de sulfatation ($SO_3^-/COO^-$): 2,8 ± 0,1.

– Spectre IR: large bande entre 1300 et 1200 $cm^{-1}$ caractéristique des groupes sulfates.

– Electrophorèse en HCl: la figure 9 montre les tracés relatifs à l'héparine de départ D-98 et au produit AH-118. On peut noter l'augmentation significative de la migration électrophorétique de AH-118 par rapport à l'héparine de départ D-98.

La figure 9 montre aussi que le composé AH-118 possède un profil photodensitométrique analogue à ceux des composés AH-16 (exemple 1, figure 1) et AH-17 (exemple 4, figure 6), alors que l'héparine de départ D-98 se montre très hétérogène et tout à fait différente des héparines de départ utilisées dans les exemples 1 et 4.

– Electrophorèse en acétate de baryum: la figure 10 montre que AH-118 a une caractéristique électrophorétique «slow-moving», à la différence de l'héparine de départ D-98 qui contient aussi bien les espèces «slow-moving» que les espèces «fast-moving». La figure 10 confirme aussi les indications de la figure 9 et, en plus, montre, d'une façon surprenante, que le composé AH-118 n'est pas significativement différent du AH-108 de l'exemple 11, bien que les héparines de départ soient profondément différentes.

**Revendications pour les Etats contractants BE, CH, DE, FR, GB, IT, LI, LU, NL, SE:**

1. Héparine dépolymérisée et supersulfatée ayant un poids moléculaire compris entre 2000 et 9000 et un degré de sulfatation d'au moins 2,5, représentée par la formule suivante:

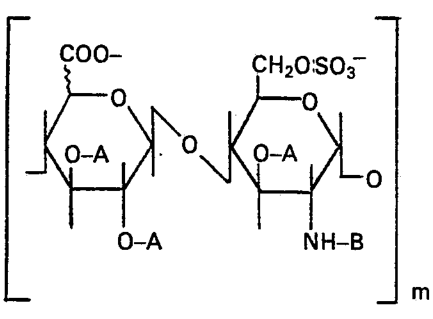

IV

dans laquelle A représente H et $SO_3^-$, B représente $SO_3^-$ et $COCH_3$ et m est compris entre 4 et 15, et ses sels pharmaceutiquement acceptables.

2. Héparine dépolymérisée et supersulfatée selon la revendication 1, sous forme de sel de sodium.

3. Héparine dépolymérisée et supersulfatée selon la revendication 1, sous forme de sel de calcium.

4. Procédé pour la préparation de l'héparine selon l'une quelconque des revendications 1 à 3, caractérisé en ce qu'une héparine d'origine naturelle, ou une de ses fractions, est traitée avec un mélange d'acide sulfurique et d'acide chlorosulfonique et le produit ainsi obtenu est isolé sous forme de sel alcalin ou transformé dans la forme acide ou dans un autre sel pharmaceutiquement acceptable.

5. Procédé selon la revendication 4, caractérisé en ce que la réaction est conduite à une température comprise entre −20 et +40 °C.

6. Procédé selon l'une des revendications 4 ou 5, caractérisé en ce que la concentration des deux acides est au moins 95% en poids.

7. Procédé selon l'une quelconque des revendications 4 à 6, caractérisé en ce que le rapport acide sulfurique: acide chlorosulfonique est d'environ 2:1.

8. Procédé selon l'une quelconque des revendications 4 à 7, caractérisé en ce que l'héparine dépolymérisée et supersulfatée est isolée sous forme de sel de sodium.

9. Procédé selon l'une quelconque des revendications 4 à 8, caractérisé en ce que l'héparine dépolymérisée et supersulfatée obtenue présente un degré de sulfatation d'au moins 20% supérieur à celui de l'héparine de départ ou de sa fraction.

10. Composition pharmaceutique à action antithrombotique potentielle, hypolipémiante et fibrinolytique sous forme de dosage unitaire comprenant de 1 à 1500 mg d'une héparine dépolymérisée et supersulfatée selon l'une quelconque des revendications 1 à 3, en mélange avec un excipient pharmaceutique.

11. Compositions pharmaceutiques pour le traitement de l'athérosclérose et la prévention des thromboses renfermant, en tant qu'ingrédient actif, une héparine dépolymérisée et supersulfatée selon l'une quelconque des revendications 1 à 3.

**Revendications pour l'Etat contractant AT:**

1. Procédé pour la préparation d'une nouvelle héparine dépolymérisée et supersulfatée ayant un poids moléculaire compris entre 2000 et 9000 et un degré de sulfatation d'au moins 2,5, représentée par la formule suivante:

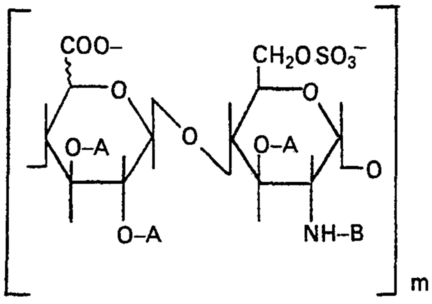

IV

dans laquelle A représente H et $SO_3^-$, B représente $SO_3^-$ et $COCH_3$ et m est compris entre 4 et 15, et de ses sels pharmaceutiquement acceptables, caractérisé en ce qu'une héparine d'origine naturelle ou une de ses fractions, est traitée avec un mélange d'acide sulfurique et d'acide chlorosulfonique et le produit ainsi obtenu est isolé sous forme de sel alcalin ou transformé dans la forme acide ou dans un autre sel pharmaceutiquement acceptable.

2. Procédé selon la revendication 1, caractérisé en ce que la réaction est conduite à une température comprise entre −20 et +40 °C.

3. Procédé selon l'une des revendications 1 ou 2, caractérisé en ce que la concentration des deux acides est au moins 95% en poids.

4. Procédé selon l'une quelconque des revendications 1 à 3, caractérisé en ce que le rapport acide sulfurique: acide chlorosulfonique est d'environ 2:1.

5. Procédé selon l'une quelconque des revendications 1 à 4, caractérisé en ce que l'héparine dépolymérisée et supersulfatée est isolée sous forme de sel de sodium.

6. Procédé selon l'une quelconque des revendications 1 à 5, caractérisé en ce que l'héparine dépolymérisée et supersulfatée obtenue présente un degré de sulfatation d'au moins 20% supérieur à celui de l'héparine de départ ou de sa fraction.

**Patentansprüche für die Vertragsstaaten BE, CH, DE, FR, GB, IT, LI, LU, NL, SE:**

1. Depolymerisiertes und supersulfatisiertes Heparin mit einem Molekulargewicht zwischen 2000 und 9000 und einem Sulfatisierungsgrad von mindestens 2,5, dargestellt durch die folgende Formel:

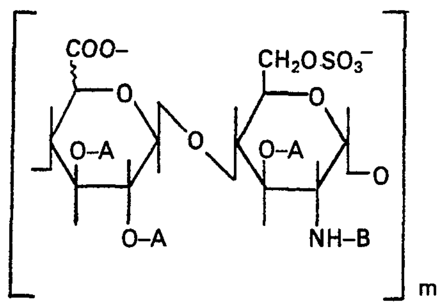

IV

worin A für H und $SO_3^-$ steht, B für $SO_3^-$ und $COCH_3$ steht und m zwischen 4 und 15 beträgt, und dessen pharmazeutisch akzeptable Salze.

2. Depolymerisiertes und supersulfatisiertes Heparin nach Anspruch 1 in Form des Natriumsalzes.

3. Depolymerisiertes und supersulfatisiertes Heparin nach Anspruch 1 in Form des Calciumsalzes.

4. Verfahren zur Herstellung des Heparins nach einem der Ansprüche 1 bis 3, dadurch gekennzeichnet, dass ein Heparin natürlichen Ursprungs oder eine seiner Fraktionen mit einer Mischung aus Schwefelsäure und Chlorsulfonsäure behandelt und das so erhaltene Produkt in Form des

Alkalisalzes isoliert oder in Säureform oder ein anderes pharmazeutisch akzeptables Salz übergeführt wird.

5. Verfahren nach Anspruch 4, dadurch gekennzeichnet, dass die Umsetzung bei einer Temperatur zwischen −20 und +40 °C erfolgt.

6. Verfahren nach einem der Ansprüche 4 oder 5, dadurch gekennzeichnet, dass die Konzentration der beiden Säuren mindestens 95 Gew.-% beträgt.

7. Verfahren nach einem der Ansprüche 4 bis 6, dadurch gekennzeichnet, dass das Verhältnis Schwefelsäure : Chlorsulfonsäure etwa 2:1 beträgt.

8. Verfahren nach einem der Ansprüche 4 bis 7, dadurch gekennzeichnet, dass das depolymerisierte und supersulfatisierte Heparin in Form des Natriumsalzes isoliert wird.

9. Verfahren nach einem der Ansprüche 4 bis 8, dadurch gekennzeichnet, dass das erhaltene depolymerisierte und supersulfatisierte Heparin einen Sulfatisierungsgrad aufweist, der mindestens 20% höher als jener des Ausgangsheparins oder seiner Fraktion ist.

10. Pharmazeutische Zusammensetzung mit potentieller antithrombotischer, hypolipämischer und fibrinolytischer Wirkung in Einheitsdosisform, umfassend 1 bis 1500 mg eines depolymerisierten und supersulfatisierten Heparins nach einem der Ansprüche 1 bis 3 in Mischung mit einem pharmazeutischen Exzipienten.

11. Pharmazeutische Zusammensetzung zur Behandlung von Arteriosklerose und Verhinderung von Thrombosen, welche als aktives Ingrediens ein depolymerisiertes und supersulfatisiertes Heparin nach einem der Ansprüche 1 bis 3 enthält.

**Patentansprüche für den Vertragsstaat AT:**

1. Verfahren zur Herstellung eines neuen depolymerisierten und supersulfatisierten Heparins mit einem Molekulargewicht zwischen 2000 und 9000 und einem Sulfatisierungsgrad von mindestens 2,5, dargestellt durch die folgende Formel:

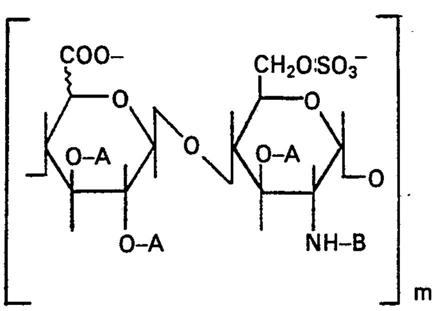

IV

worin A für H und $SO_3^-$ steht, B für $SO_3^-$ und $COCH_3$ steht und m zwischen 4 und 15 beträgt, und seiner pharmazeutisch akzeptablen Salze, dadurch gekennzeichnet, dass ein Heparin natürlichen Ursprungs oder eine seiner Fraktionen mit einer Mischung aus Schwefelsäure und Chlorsulfonsäure behandelt und das so erhaltene Produkt in Form des Alkalisalzes isoliert oder in Säureform oder ein anderes pharmazeutisch akzeptables Salz übergeführt wird.

2. Verfahren nach Anspruch 1, dadurch gekennzeichnet, dass die Umsetzung bei einer Temperatur zwischen −20 und +40 °C erfolgt.

3. Verfahren nach einem der Ansprüche 1 oder 2, dadurch gekennzeichnet, dass die Konzentration der beiden Säuren mindestens 95 Gew.-% beträgt.

4. Verfahren nach einem der Ansprüche 1 bis 3, dadurch gekennzeichnet, dass das Verhältnis Schwefelsäure:Chlorsulfonsäure etwa 2:1 beträgt.

5. Verfahren nach einem der Ansprüche 1 bis 4, dadurch gekennzeichnet, dass das depolymerisierte und supersulfatisierte Heparin in Form des Natriumsalzes isoliert wird.

6. Verfahren nach einem der Ansprüche 1 bis 5, dadurch gekennzeichnet, dass das erhaltene depolymerisierte und supersulfatisierte Heparin einen Sulfatisierungsgrad aufweist, der mindestens 20% höher als jener des Ausgangsheparins oder seiner Fraktion ist.

**Claims for the contracting states BE, CH, DE, FR, GB, IT, LI, LU, NL, SE:**

1. A depolymerized and supersulfated heparin having a molecular weight between 2000 and 9000 and a sulfatation degree of at least 2,5, represented by the following formula

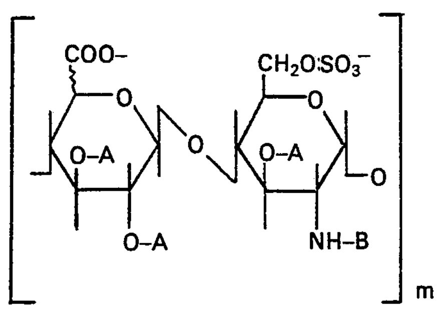

IV

wherein A represents H and $SO_3^-$, B represents $SO_3^-$ and $COCH_3$ and m varies between 4 and 15, and the pharmaceutically acceptable salts thereof.

2. A depolymerized and supersulfated heparin according to claim 1, in the form of sodium salt.

3. A depolymerized and supersulfated heparin according to claim 1, in the form of calcium salt.

4. A process for the preparation of the heparin according to any one of claims 1 to 3, characterized in that a heparin of natural origin or one of the fractions thereof is treated with a mixture of sulfuric acid and chlorosulfonic acid and the product thus obtained is isolated in the form of an alkali metal salt or converted into the acid form or into another pharmaceutically acceptable salt.

5. A process according to claim 4, characterized in that the reaction is carried out at a temperature between −20 and +40 °C.

6. A process according to any one of claims 4 or 5, characterized in that the concentration of the two acids is at least 95% by weight.

7. A process according to any one of claims 4 to 6, characterized in that the ratio sulfuric acid: chlorosulfonic acid is about 2:1.

8. A process according to any one of claims 4 to 7, characterized in that the depolymerized and supersulfated heparin is isolated in the form of sodium salt.

9. A process according to any one of claims 4 to 8, characterized in that the depolymerized and supersulfated heparin obtained has a sulfatation degree at least 20% higher than that of the starting heparin or a fraction thereof.

10. A pharmaceutical composition having potential antithrombotic action and hypolipemic and fibrinolytic activity in dosage unit form comprising from 1 to 1500 mg of a depolymerized and supersulfated heparin according to any one of claims 1 to 3 in admixture with a pharmaceutical excipient.

11. A pharmaceutical composition for the treatment of atherosclerosis and the prevention of thrombosis, comprising, as active ingredient, a depolymerized and supersulfated heparin according to any one of claims 1 to 3.

**Claims for the contracting state AT:**

1. A process for the preparation of a new depolymerized and supersulfated heparin having a molecular weight between 2000 and 9000 and a sulfatation degree of at least 2,5, represented by the following formula

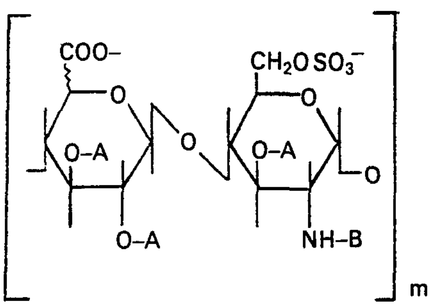

IV

wherein A represents H and $SO_3^-$, B represents $SO_3^-$ and $COCH_3$ and m varies between 4 and 15, and of the pharmaceutically acceptable salts thereof, characterized in that a heparin of natural origin or one of the fractions thereof is treated with a mixture of sulfuric acid and chlorosulfonic acid and the product thus obtained is isolated in the form of an alkali metal salt or converted into the acid form or into another pharmaceutical acceptable salt.

2. A process according to claim 1, characterized in that the reaction is carried out at a temperature between −20 and +40 °C.

3. A process according to any one of claims 1 or 2, characterized in that the concentration of the two acids is at least 95% by weight.

4. A process according to any one of claims 1 to 3, characterized in that the ratio sulfuric acid: chlorosulfonic acid is about 2:1.

5. A process according to any one of claims 1 to 4, characterized in that the depolymerized and supersulfated heparin is isolated in the form of sodium salt.

6. A process according to any one of claims 1 to 5, characterized in that the depolymerized and supersulfated heparin obtained has a sulfatation degree at least 20% higher than that of the starting heparin of a fraction thereof.

Fig-1

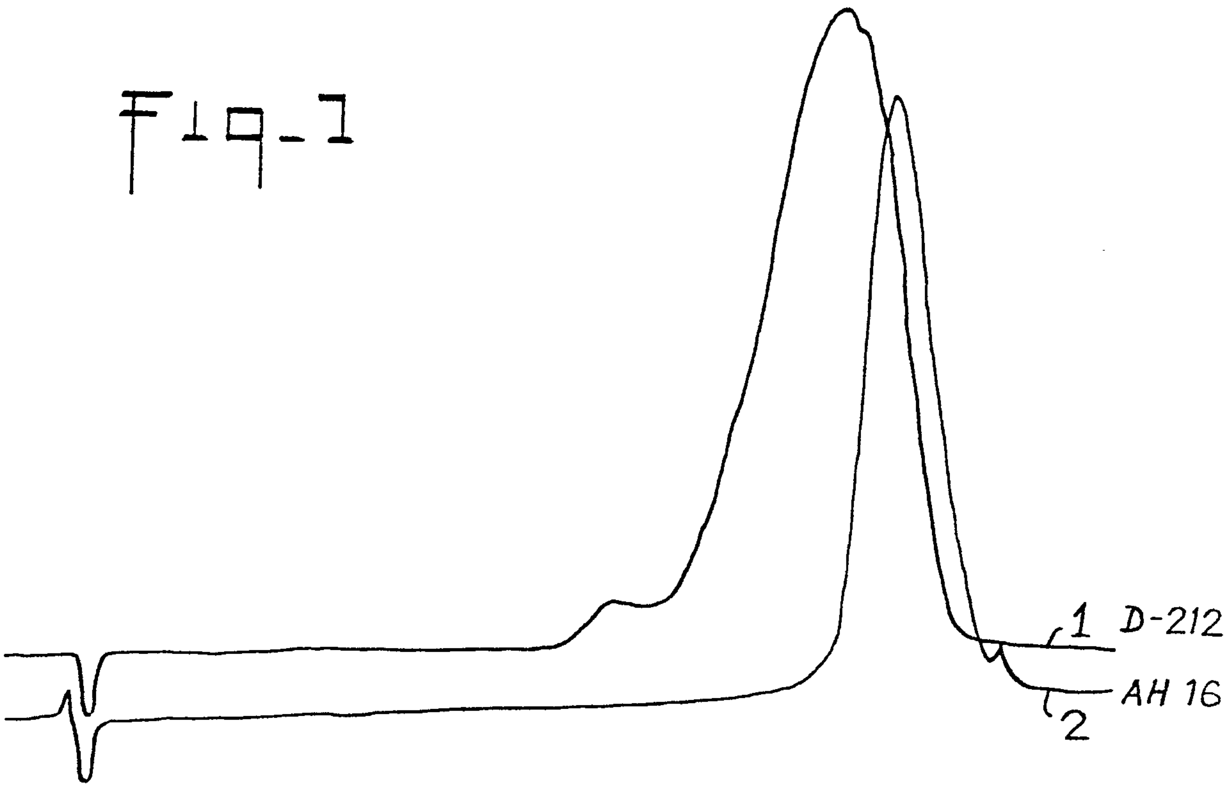


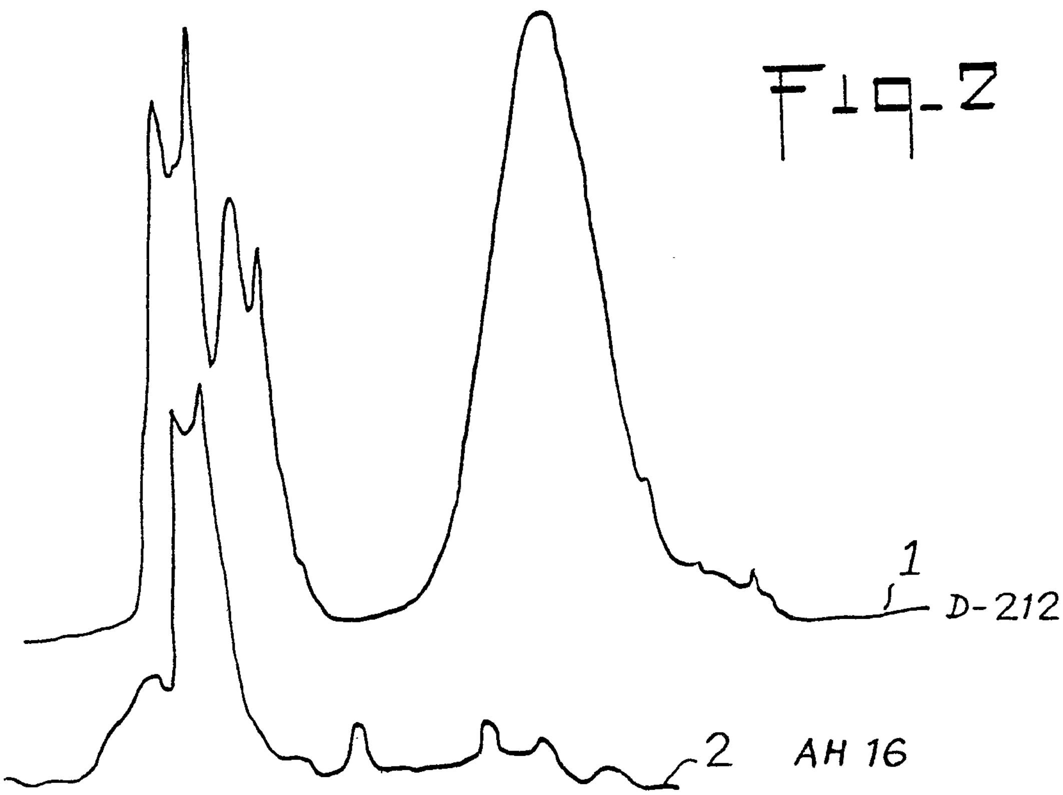


Fig-2

Fig-3

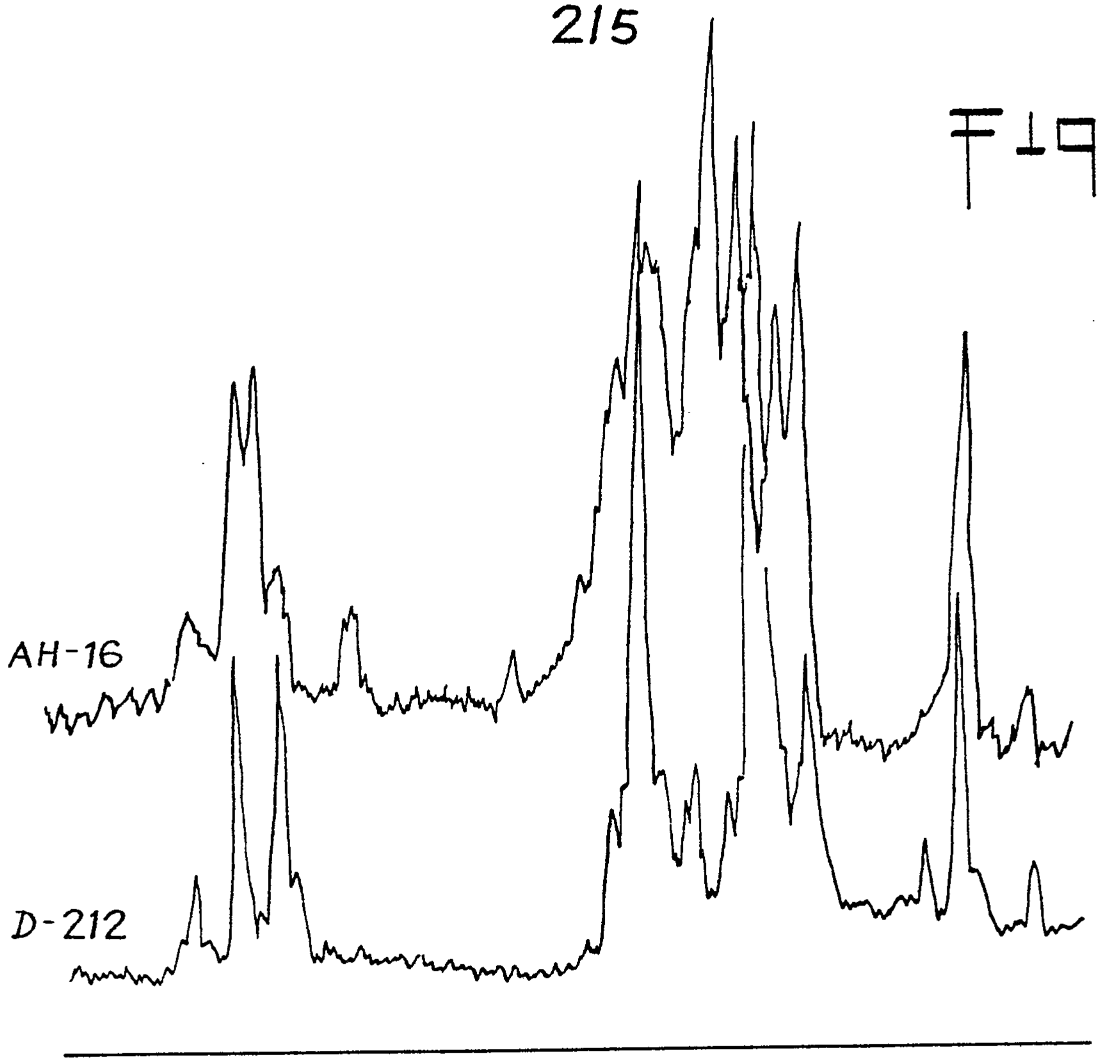


Fig-6

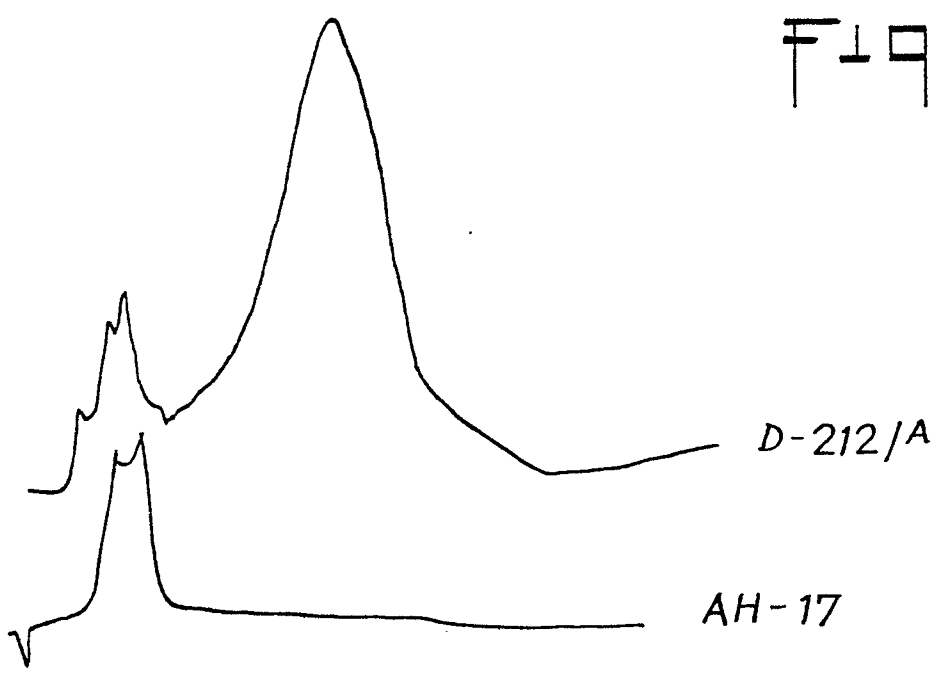

Fig-4

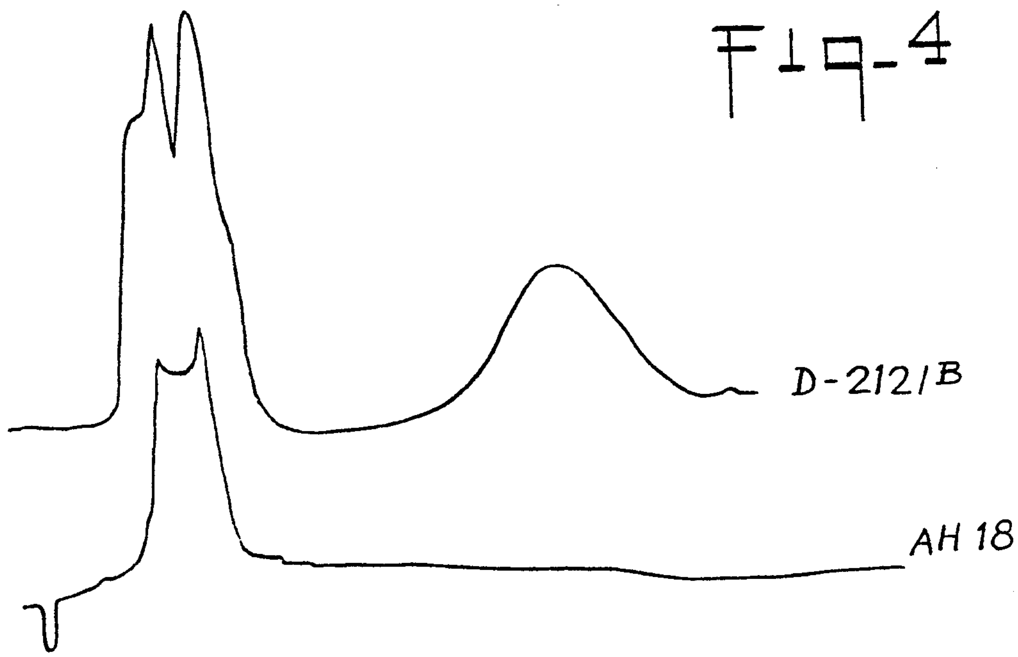


Fig-5

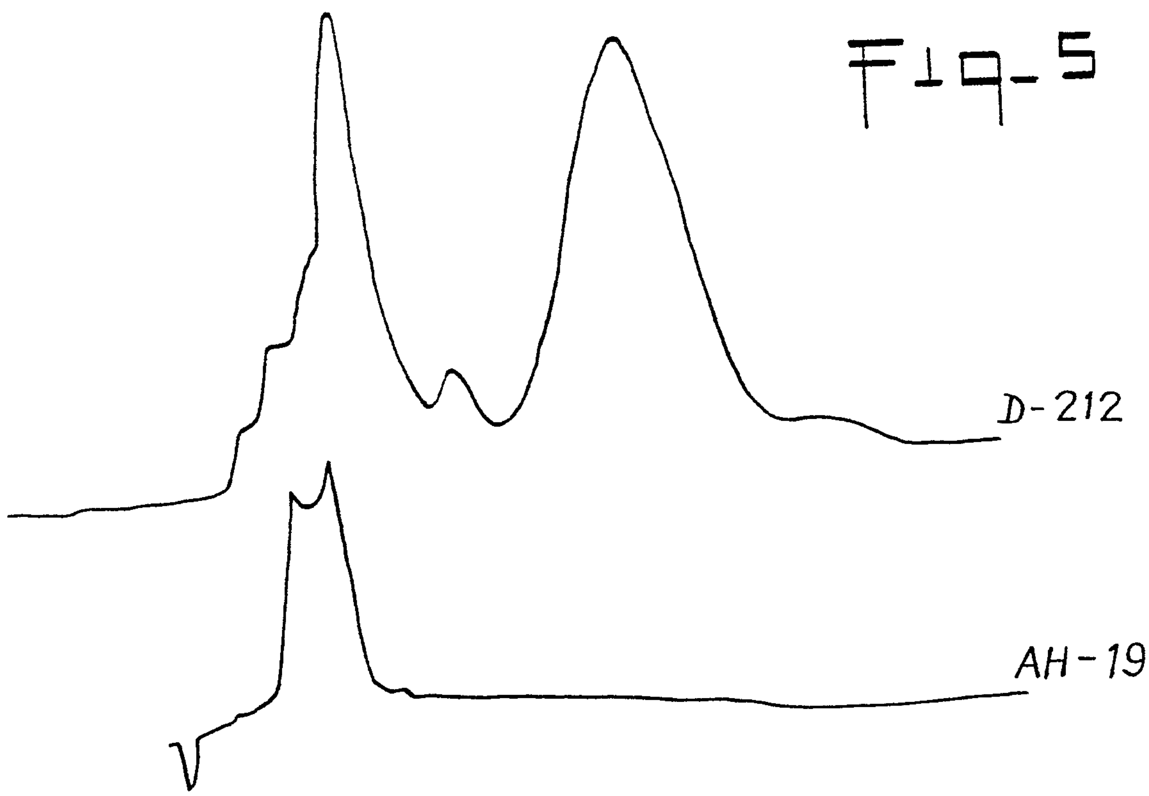

Fig-7

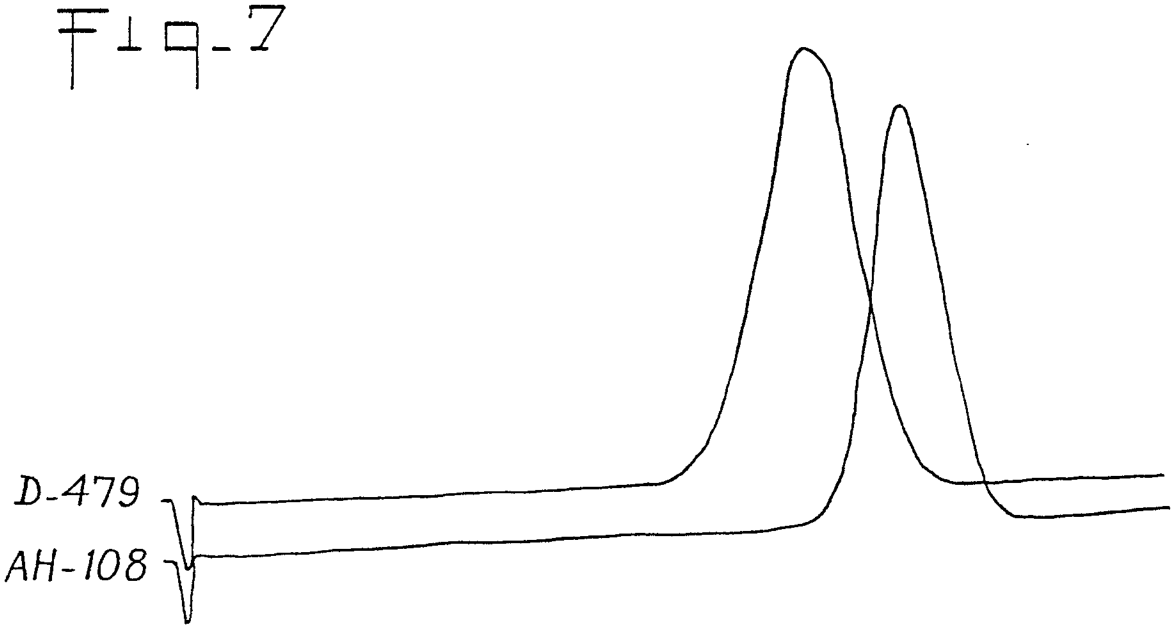


Fig-8

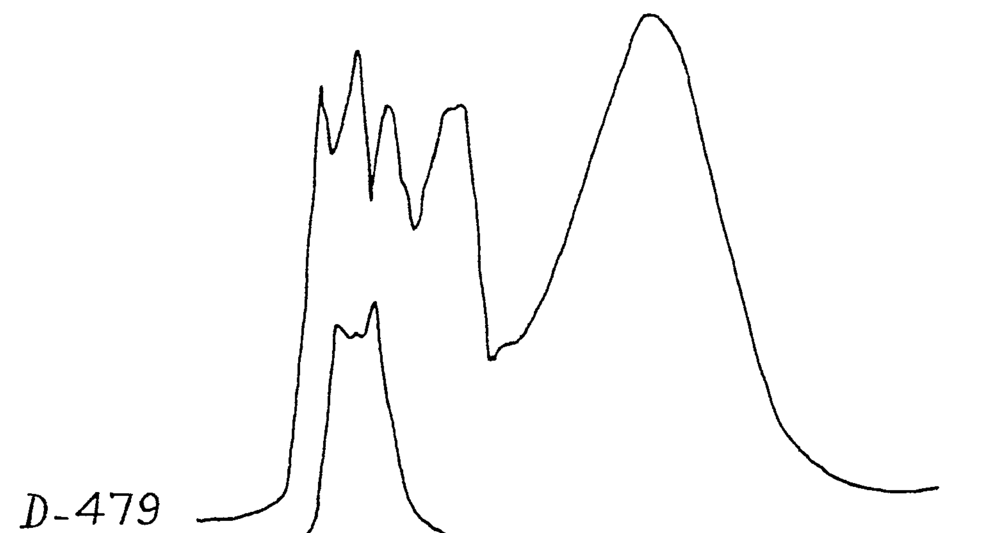

Fig-9

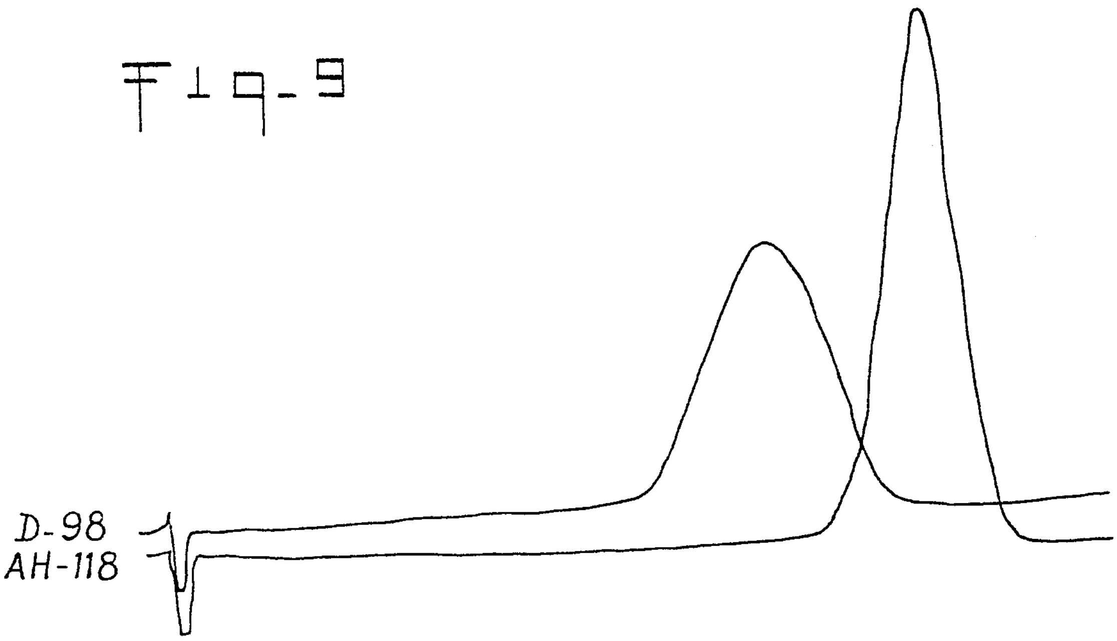


Fig-10